# EUROPEAN PATENT SPECIFICATION

(11) **EP 2 211 859 B1**
(45) Date of publication and mention of the grant of the patent: **06.01.2016**
(21) Application number: 08805185.9
(22) Date of filing: 09.10.2008
(51) Int. Cl.: A61K 31/404, A61K 31/198, A61P 25/16, A61P 25/14

(54) **METABOTROPIC GLUTAMATE RECEPTOR MODULATORS FOR THE TREATMENT OF PARKINSON'S DISEASE**
METABOTROPE GLUTAMAT-REZEPTOR-MODULATOREN ZUR BEHANDLUNG VON PARKINSON-KRANKHEIT
MODULATEURS DES RÉCEPTEURS METABOTROPIQUES DU GLUTAMATE POUR LE TRAITEMENT DE LA MALADIE DE PARKINSON

(30) Priority: 12.10.2007 US 979486 P; 05.05.2008 US 50333 P
(43) Date of publication of application: 04.08.2010
(73) Proprietor: Novartis AG, 4056 Basel (CH)
(72) Inventor: UMBRICHT, Daniel, CH-8037 Zurich (CH); GOMEZ-MANCILLA, Baltazar, CH-4051 Basel (CH); GASPARINI, Fabrizio, CH-4415 Lausen (CH); DI PAOLO, Thérése, Québec,Qc G1V 4G2 (CA)
(74) Representative: Jeffries, Charles Edward
(86) International application number: PCT/EP2008/063544
(87) International publication number: WO 2009/047296

(56) References cited:
- WO-A-00/73283
- WO-A-03/047581
- WO-A-2004/024074
- WO-A-2005/030128
- WO-A-2005/079802
- WO-A-2006/094639
- WO-A-2006/114260
- WO-A-2006/114262
- WO-A-2007/021575
- WO-A-2007/071358
- WO-A-2008/031550
- OSSOWSKA K ET AL: "Blockade of the metabotropic glutamate receptor subtype 5 (mG1uR5) produces antiparkinsonian-like effects in rats" NEUROPHARMACOLOGY, vol. 41, no. 4, 1 September 2001 (2001-09-01), pages 413-420, XP002986198 ISSN: 0028-3908
- OSSOWSKA K ET AL: "An influence of ligands of metabotropic glutamate receptor subtypes on parkinsonian-like symptoms and the striatopallidal pathway in rats." AMINO ACIDS, vol. 32, no. 2, February 2007 (2007-02), pages 179-188, XP002516005 ISSN: 0939-4451
- VERNON A C ET AL: "Neuroprotective effects of metabotropic glutamate receptor ligands in a 6-hydroxydopamine rodent model of Parkinson's disease." THE EUROPEAN JOURNAL OF NEUROSCIENCE, vol. 22, no. 7, October 2005 (2005-10), pages 1799-1806, XP002516017 ISSN: 0953-816X
- DEKUNDY A ET AL: "Effects of group I metabotropic glutamate receptors blockade in experimental models of Parkinson's disease", BRAIN RESEARCH BULLETIN, ELSEVIER SCIENCE LTD, OXFORD, GB, vol. 69, no. 3, 14 April 2006 (2006-04-14) , pages 318-326, XP024900757, ISSN: 0361-9230, DOI: 10.1016/J.BRAINRESBULL.2005.12.009 [retrieved on 2006-04-14]
- HILL M P ET AL: "The mGlu5 receptor antagonist SIB-1830 reduces L-DOPA-induced dyskinesia in the MPTP-lesioned primate model of Parkinson's disease", SOCIETY FOR NEUROSCIENCE ABSTRACTS, vol. 27, no. 1, November 2001 (2001-11), page 533, & 31ST ANNUAL MEETING OF THE SOCIETY FOR NEUROSCIENCE; SAN DIEGO, CALIFORNIA, USA; NOVEMBER 10-15 2001 ISSN: 0190-5295

## Description

The invention is set out in the appended claims.

This disclosure relates to new pharmaceutical uses of compounds acting as modulators of metabotropic glutamate receptors ("mGluR modulators"), including antagonists of metabotropic glutamate receptors ("mGluR antagonists"). In particular, the disclosure relates to new uses of antagonists of metabotropic glutamate type-5 receptors ("mGluR5 antagonists").

WO 2005/079802, WO 2003/047581, WO 2004/000316, WO 2005/044265, WO 2005/044266, WO 2005/044267, WO 2006/114262 and WO 2007/071358 disclose mGluR5 antagonists and their use as pharmaceuticals.

Dekundy et al., Brain Research Bulletin 69(3), pages 318-326 (2006), evaluated antidyskinetic activity of MTEP, an mGluR5 antagonist, in a rat model. MTEP inhibited contralateral rotations after L-DOPA and alleviated L-DOPA induced dyskinesia, suggesting that MTEP might reduce antiparkinsonian actions of L-DOPA. Hill et al., Society Neuroscience Abstracts 27(1), page 833 (2001) report that SIB-1830, an mGluR5 antagonist, reduced in a dose-dependent manner the L-DOPA induced dyskinesia in the MPTP-lesioned primate model of Parkinson's disease.

It has been surprisingly found that compounds having mGluR modulating activity, in particular antagonistic activity, may be used to treat Parkinson's Disease and disorders associated with Parkinson's Disease. In particular, it has been found that mGluR modulators may be used to treat dyskinesia, a disorder associated with Parkinson's Disease and treatment thereof. In particular, it has been found that mGluR5 modulators, e.g. mGluR5 antagonists, may be used to treat Parkinson's Disease and associated disorders e.g. Parkinson's dyskinesia, for example, Parkinson's Disease levodopa (L-dopa) induced Parkinson's dyskinesia.

Accordingly, a first aspect of the disclosure concerns an mGluR modulator for use in the treatment (whether therapeutic or prophylactic), prevention and/or delay of progression of Parkinson's Disease and/or disorders associated therewith. In one embodiment, the disclosure concerns an mGluR modulator e.g. an antagonist, for use in the treatment, prevention and/or delay of progression of Parkinson's dyskinesia, for example, Parkinson's Disease levodopa (L-dopa) induced dyskinesia (PD-LID).

A further aspect of the disclosure relates to a pharmaceutical composition comprising an mGluR, e.g. mGluR5, modulator for the treatment, prevention or delay of progression of Parkinson's Disease and/or disorders associated with Parkinson's disease. In one embodiment, the composition is for the treatment, prevention or delay of progression of Parkinson's dyskinesia e.g. Parkinson's Disease levodopa (L-dopa) induced dyskinesia (PD-LID). In one embodiment, the pharmaceutical composition is for the treatment, prevention or delay of progression of Parkinson's Disease.

A further aspect of the disclosure relates to the use of an mGluR, e.g. mGluR5, modulator for the manufacture of a medicament for the treatment, prevention or delay of progression of Parkinson's Disease and/or disorders associated with Parkinson's disease. In one embodiment, the medicament is for the treatment, prevention or delay of progression of Parkinson's dyskinesia e.g. Parkinson's Disease levodopa (L-dopa) induced dyskinesia (PD-LID).

The mGluR modulator may be an mGluR5 modulator. In certain embodiments, the mGluR modulator is an mGluR, e.g. mGluR5, antagonist.

In the present specification, the following definitions shall apply if no specific other definition is given:
"Alkyl" represents a straight-chain or branched-chain alkyl group, preferably represents a straight-chain or branched-chain C₁₋₁₂ alkyl, particularly preferably represents a straight-chain or branched-chain C₁₋₆ alkyl; for example, methyl, ethyl, n- or iso-propyl, n-, iso-, sec- or tert-butyl, n-pentyl, n-hexyl, n-heptyl, n-octyl, n-nonyl, n-decyl, n-undecyl, n-dodecyl, with particular preference given to methyl, ethyl, n-propyl and iso-propyl.
"Alkandiyl" represents a straight-chain or branched-chain alkandiyl group bound by two different carbon atoms to the molecule, it preferably represents a straight-chain or branched-chain C₁₋₁₂ alkandiyl, particularly preferably represents a straight-chain or branched-chain C₁₋₆ alkandiyl; for example, methandiyl (-CH₂-), 1,2-ethanediyl (-CH₂-CH₂-), 1,1-ethanediyl ((-CH(CH₃)-), 1,1-, 1,2-, 1,3-propanediyl and 1,1-, 1,2-, 1,3-, 1,4-butanediyl, with particular preference given to methandiyl, 1,1-ethanediyl, 1,2-ethanediyl, 1,3-propanediyl or 1,4-butanediyl.

Each alkyl part of "alkoxy", "alkoxyalkyl", "alkoxycarbonyl", "alkoxycarbonylalkyl" and "halogenalkyl" shall have the same meaning as described in the above-mentioned definition of "alkyl".

"Alkenyl" represents a straight-chain or branched-chain alkenyl group, preferably C₂₋₆ alkenyl, for example, vinyl, allyl, 1-propenyl, isopropenyl, 2-butenyl, 2-pentenyl, 2-hexenyl, etc. and preferably represents C₂₋₄ alkenyl.

"Alkendiyl" represents a straight-chain or branched-chain alkendiyl group bound by two different carbon atoms to the molecule, it preferably represents a straight-chain or branched-chain C₂₋₆ alkandiyl; for example, -CH=CH-, -CH=C(CH₃)-, -CH=CH-CH₂-, -C(CH₃)=CH-CH₂-, -CH=C(CH₃)-CH₂-, -CH=CH-C(CH₃)H-, -CH=CH-CH=CH-, -C(CH₃)=CH-CH=CH-, -CH=C(CH₃)-CH=CH-, with particular preference given to -CH=CH-CH₂-, -CH=CH-CH=CH-.

"Alkynyl" represents a straight-chain or branched-chain alkynyl group, preferably C₂₋₆ alkynyl, for example, ethenyl, propargyl, 1-propynyl, isopropenyl, 1- (2- or 3) butynyl, 1- (2- or 3) pentenyl, 1- (2- or 3) hexenyl, etc., preferably represents C₂₋₄ alkynyl and particularly preferably represents ethynyl.

"Aryl" represents an aromatic hydrocarbon group, preferably a C₆₋₁₀ aromatic hydrocarbon group; for example phenyl, naphthyl, especially phenyl.

"Aralkyl" denotes an "aryl" bound to an "alkyl" (both as defined above) an represents, for example benzyl, α-methylbenzyl, 2-phenylethyl, α,α-dimethylbenzyl, especially benzyl.

"Heterocycle" represents a saturated, partly saturated or aromatic ring system containing at least one hetero atom. Preferably, heterocycles consist of 3 to 11 ring atoms of which 1-3 ring atoms are hetero atoms. Heterocycles may be present as a single ring system or as bicyclic or tricyclic ring systems; preferably as single ring system or as benz-annelated ring system. Bicyclic or tricyclic ring systems may be formed by annelation of two or more rings, by a bridging atom, e.g. oxygen, sulfur, nitrogen or by a bridging group, e.g. alkandiyl or alkenediyl. A heterocycle may be substituted by one or more substituents selected from the group consisting of oxo (=O), halogen, nitro, cyano, alkyl, alkandiyl, alkenediyl, alkoxy, alkoxyalkyl, alkoxycarbonyl, alkoxycarbonylalkyl, halogenalkyl, aryl, aryloxy and arylalkyl. Examples of heterocyclic moieties include pyrrole, pyrroline, pyrrolidine, pyrazole, pyrazoline, pyrazolidine, imidazole, imidazoline, imidazolidine, triazole, triazoline, triazolidine, tetrazole, furane, dihydrofurane, tetrahydrofurane, furazane (oxadiazole), dioxolane, thiophene, dihydrothiophene, tetrahydrothiophene, oxazole, oxazoline, oxazolidine, isoxazole, isoxazoline, isoxazolidine, thiazole, thiazoline, thiazlolidine, isothiazole, istothiazoline, isothiazolidine, thiadiazole, thiadiazoline, thiadiazolidine, pyridine, piperidine, pyridazine, pyrazine, piperazine, triazine, pyrane, tetrahydropyrane, thiopyrane, tetrahydrothiopyrane, oxazine, thiazine, dioxine, morpholine, purine, pterine, and the corresponding benz-annelated heterocycles, e.g. indole, isoindole, cumarine, cumaronecinoline, isochinoline and cinnoline.

"Hetero atoms" are atoms other than carbon and hydrogen, preferably nitrogen (N), oxygen (O) or sulfur (S).

"Halogen" represents fluoro, chloro, bromo or iodo, preferably represents fluoro, chloro or bromo and particularly preferably represents chloro.

Various compounds having mGluR, in particular mGluR5, modulating activity are described herein. Where the specification refers to compounds, agents or active ingredients of the disclosure this is generally taken to mean a compound having mGluR modulating activity unless specified otherwise. In embodiments of the disclosure the mGluR modulators are mGluR5 antagonists. When the specification refers to mGluR antagonists, this is generally taken to include compounds that are capable of interacting with an mGluR to inhibit the effect of a natural ligand for the mGluR e.g. such that a response pathway of a mGluR expressing cell is not stimulated.

In one embodiment, the mGluR modulator is a mGluR5 antagonist.

Compounds of the disclosure may exist in free or acid addition salt form. In this specification, unless otherwise indicated, reference to "compounds of the disclosure" is to be understood as embracing the compounds in any form, for example free base or acid addition salt form. Salts which are unsuitable for pharmaceutical uses but which can be employed, for example, for the isolation or purification of free compounds of the disclosure, such as picrates or perchlorates, are also included. For therapeutic use, only pharmaceutically acceptable salts or free compounds are employed (where applicable in the form of pharmaceutical preparations), and are therefore preferred.

It will be understood that any discussion of methods or references to the active ingredients also includes pharmaceutically acceptable salts. If these active ingredients have, for example, at least one basic center, they can form acid addition salts. Corresponding acid addition salts can also be formed having, if desired, an additionally present basic center. The active ingredients having an acid group (for example COOH) can also form salts with bases. The active ingredient or a pharmaceutically acceptable salt thereof may also be used in the form of a hydrate or may include other solvents used for crystallization. Examples of mGluR5 modulators, e.g. antagonists, and their manufacture are known, e.g. from WO 03/047581 and WO 2006/11 4262.

On account of the asymmetrical carbon atom(s) that may be present in the compounds of the disclosure and their salts, the compounds may exist in optically active form or in form of mixtures of optical isomers, e.g. in form of racemic mixtures or diastereomeric mixtures. All optical isomers and their mixtures, including racemic mixtures, are part of the present disclosure.

In another embodiment, the mGluR modulator is a compound of the formula (IV): 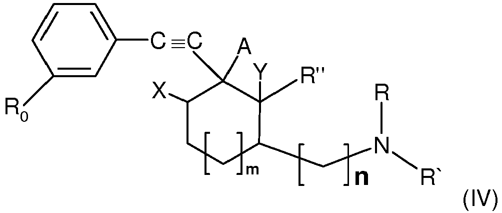 wherein
m is 0 or 1,
n is 0 or 1 and
A is hydroxy
X is hydrogen and
Y is hydrogen, or
A forms a single bond with X or with Y;
R₀ is hydrogen, (C₁₋₄)alkyl, (C₁₋₄)alkoxy, trifluoromethyl, halogen, cyano, nitro, -COOR₁ wherein R₁ is (C₁₋₄)alkyl or -COR₂ wherein R₂ is hydrogen or (C₁₋₄)alkyl, and 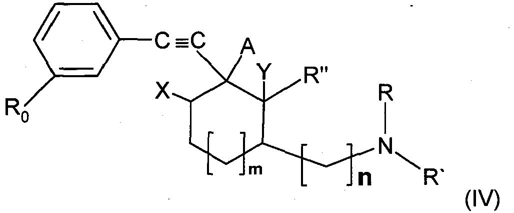 wherein
   m is 0 or 1,
   n is 0 or 1 and
   A is hydroxy
   X is hydrogen and
   Y is hydrogen, or
   A forms a single bond with X or with Y;
   R₀ is hydrogen, (C₁₋₄)alkyl, (C₁₋₄)alkoxy, trifluoromethyl, halogen, cyano, nitro, -COOR₁ wherein R₁ is (C₁₋₄)alkyl or -COR₂ wherein R₂ is hydrogen or (C₁₋₄)alkyl, and R is -COR₃, -COOR₃, -CONR₄R₅ or -SO₂R₆, wherein R₃ is (C₁₋₄)alkyl, (C₃₋₇)cycloalkyl or optionally substituted phenyl, 2-pyridyl or 2-thienyl; R₄ and R₅, independently, are hydrogen or (C₁₋₄)alkyl; and R₆ is (C₁₋₄)alkyl, (C₃₋₇)cycloalkyl or optionally substituted phenyl,
   R' is hydrogen or (C₁₋₄)alkyl and
   R" is hydrogen or (C₁₋₄)alkyl, or
   R' and R" together form a group -CH₂-(CH₂)ₘ-
   wherein m is 0, 1 or 2, in which case one of n and m is different from 0,
   with the proviso that R₀ is different from hydrogen, trifluoromethyl and methoxy when n is 0, A is hydroxy, X and Y are both hydrogen, R is COOEt and R' and R" together form a group-(CH₂)₂-,
   in free base or acid addition salt form.

Exemplary compounds of formula (IV) include:
(-)-(3aR,4S,7aR)-4-Hydroxy-4-m-tolylethynyl-octahydro-indole-1-carboxylic acid methyl ester
(-)-(3aR,4S,7aR)-4-Hydroxy-4-m-tolylethynyl-octahydro-indole-1-carboxylic acid ethyl ester
(-)-(3aR,4S,7aR)-Furan-2-yl-(4-hydroxy-4-m-tolylethynyl-octahydro-indol-1-yl)-methanone
(±)- (3aRS,4SR,7aRS)-4-(3-Chlorophenylethynyl)-4-hydroxy-octahydro-indole-1-carboxylic acid ethyl ester
(±)-(3aRS,4SR,7aRS)-4-(3-Fluoro-phenylethynyl)-4-hydroxy-octahydro-indole-1-carboxylic acid ethyl ester
(3aRS,4SR,7aRS)-4-Hydroxy-4-phenylethynyl-octahydro-indole-1-carboxylic acid(S)(tetrahydrofuran-3-yl)ester
(3aRS,4SR,7aRS)-4-Hydroxy-4-phenylethynyl-octahydro-indole-1-carboxylic acid(R)(tetrahydrofuran-3-yl)ester
(3aRS,4SR,7aRS)-4-Hydroxy-4-(3-chlorophenylethynyl)-octahydro-indol-1-carboxylic acid-(S)(tetrahydrofuran-3yl)ester
(±)-(3aRS,4SR,7aRS)-4-Hydroxy-4-m-tolylethynyl-octahydro-indole-1-carboxylic acid ethyl ester
(±)-(3aRS,4SR,7aRS)-4-(4-Fluoro-phenylethynyl)-4-hydroxy-octahydro-indole-1-carboxylic acid ethyl ester
(±)-(3aRS,4SR,7aRS)-4-(3-chlorophenylethynyl)-4-hydroxy-1-methanesulfonyl-octahydro-indole
(±)-(3aRS,7aRS)-4-Phenylethynyl-2,3,3a,6,7,7a-hexahydro-indole-1-carboxylic acid ethyl ester and (±)-(RS)-4-phenylethynyl-2,3,5,6,7,7a-hexahydro-indole-1-carboxylic acid ethyl ester
(±)-(3RS,7aRS)-2,2,2-Trifluoro-1-(4-phenylethynyl-2,3,3a,6,7,7a-hexahydro-indol-1-yl)-ethanone
(±)-(RS)-4-m-Tolylethynyl-2,3,5,6,7,7a-hexahydro-indole-1-carboxylic acid ethyl ester (±)-(3RS,7aRS)-4-*m*-Tolylethynyl-2,3,3a,6,7,7a-hexahydro-indole-1-carboxylic acid ethyl ester
(±)-(3RS,7aRS)-4-(4-Chloro-phenylethynyl)-2,3,3a,6,7,7a-hexahydro-indole-1-carboxylic acid ethyl ester
(±)-(3RS,7aRS)-4-(2-Fluoro-phenylethynyl)-2,3,3a,6,7,7a-hexahydro-indole-1-carboxylic acid ethyl ester
(±)-(3RS,7aRS)-4-(3-Fluoro-phenylethynyl)-2,3,3a,6,7,7a-hexahydro-indole-1-carboxylic acid ethyl ester
(±)-(RS)-4-(3-Fluoro-phenylethynyl)-2,3,5,6,7,7a-hexahydro-indole-1-carboxylic acid ethyl ester
(±)-(3RS,7aRS)-4-(3-Methoxy-phenylethynyl)-2,3,3a,6,7,7a-hexahydro-indole-1-carboxylic acid ethyl ester
(±)-(RS)-4-(3-Methoxy-phenylethynyl)-2,3,5,6,7,7a-hexahydro-indole-1-carboxylic acid ethyl ester
(±)-(3aRS,4RS,7aSR)-4-Hydroxy-4-phenylethynyl-octahydro-isoindole-2-carboxylic acid ethyl ester
(±)-(3aRS,4RS,7aSR)-4-Hydroxy-4-m-tolylethynyl-octahydro-isoindole-2-carboxylic acid ethyl ester
(±)-(3aRS,4RS,7aSR)-4-Hydroxy-4-p-tolylethynyl-octahydro-isoindole-2-carboxylic acid ethyl ester
(±)-(3aRS,4RS,7aSR)-4-(3-Cyano-phenylethynyl)-4-hydroxy-octahydro-isoindole-2-carboxylic acid ethyl ester
(±)-(3aRS,4RS,7aSR)-4-Hydroxy-4-(3-methoxy-phenylethynyl)-octahydro-isoindole-2-carboxylic acid ethyl ester
(±)-(3aRS,4RS,7aSR)-4-(3-Fluoro-phenylethynyl)-4-hydroxy-octahydro-isoindole-2-carboxylic acid ethyl ester
(±)-(3aRS,4RS,7aSR)-4-Hydroxy-4-phenylethynyl-octahydro-isoindole-2-carboxylic acid *tert-*butyl ester
(±)-(3aRS,4RS,7aSR)-4-Hydroxy-4-m-tolylethynyl-octahydro-isoindole-2-carboxylic acid *tert-*butyl ester
(±)-(3aRS,4RS,7aSR)-4-Hydroxy-4-m-tolylethynyl-octahydro-isoindole-2-carboxylic acid methyl ester
(±)-(3aRS,4RS,7aSR)-Furan-2-yl-(4-hydroxy-4-m-tolylethynyl-octahydro-isoindol-2-yl)-methanone
(±)-(3aRS,4RS,7aSR)-Cyclopropyl-(4-hydroxy-4-m-tolylethynyl-octahydro-isoindol-2-yl)-methanone
(±)-(3aRS,4RS,7aSR)- (4-Hydroxy-4-m-tolylethynyl-octahydro-isoindol-2-yl)-pyridin-3-yl-methanone
(±)-((1SR,3SR)-3-Hydroxy-3-*m*-tolylethynyl-cyclohexyl)-methyl-carbamic acid methyl ester and (±)-((1RS,3SR)-3-hydroxy-3-*m*-tolylethynyl-cyclohexyl)-methyl-carbamic acid methyl ester
(±)-(1RS,3SR)-((3-Hydroxy-3-*m*-tolylethynyl-cyclohexyl)-(4-methoxy-benzyl)-carbamic acid ethyl ester
(±)-(1RS,3RS)-((3-Hydroxy-3-*m*-tolylethynyl-cyclohexyl)-(4-methoxy-benzyl)-carbamic acid ethyl ester
(±)-[(1RS,3SR)-3-Hydroxy-3-(3-methoxy-phenylethynyl)-5,5-dimethyl-cyclohexyl]-methyl-carbamic acid methyl ester
(±)-(1RS,3SR)-(3-Hydroxy-5,5-dimethyl-3-*m*-tolylethynyl-cyclohexyl)-methyl-carbamic acid methyl ester
(±)-[(1RS,3SR)-3-(3-Fluoro-phenylethynyl)-3-hydroxy-5,5-dimethyl-cyclohexyl]-methyl-carbamic acid methyl ester
(±)-[(1RS,3RS)-3-(3-Fluoro-phenylethynyl)-3-hydroxy-cyclohexyl]-methyl-carbamic acid methyl ester
(±)-[(1RS,3SR)-3-(3-Fluoro-phenylethynyl)-3-hydroxy-cyclohexyl]-methyl-carbamic acid methyl ester
(±)-[(1RS,3RS)-3-Hydroxy-3-(3-methoxy-phenylethynyl)-cyclohexyl]-methyl-carbamic acid methyl ester
(±)-[(1RS,3SR)-3-Hydroxy-3-(3-methoxy-phenylethynyl)-cyclohexyl]-methyl-carbamic acid methyl ester
(±)-[(1RS,3RS)-3-(3-Chloro-phenylethynyl)-3-hydroxy-cyclohexyl]-methyl-carbamic acid methyl ester
(±)-[(1RS,3SR)-3-(3-Chloro-phenylethynyl)-3-hydroxy-cyclohexyl]-methyl-carbamic acid methyl ester
(±)-(1RS,3RS)-N-(3-hydroxy-3-m-tolylethynyl-cyclohexyl)-acetamide
(±)-(1RS,3SR)-N-(3-hydroxy-3-m-tolylethynyl-cyclohexyl)-acetamide
(±)-(1RS,3RS)-(3-Hydroxy-3-m-tolylethynyl-cyclohexyl)-carbamic acid ethyl ester
(±)-(1RS,3SR)-(3-Hydroxy-3-m-tolylethynyl-cyclohexyl)-carbamic acid ethyl ester
(±)-(1RS,3RS)-[3-(3-Fluoro-phenylethynyl)-3-hydroxy-cyclohexyl]-carbamic acid ethyl ester
(±)-(1RS,3SR)-[3-(3-Fluoro-phenylethynyl)-3-hydroxy-cyclohexyl]-carbamic acid ethyl ester
(±)-(1RS,3RS)-[3-(3-Methoxy-phenylethynyl)-3-hydroxy-cyclohexyl]-carbamic acid ethyl ester
(±)-(1RS,3RS)-N-[3-(3-Fluoro-phenylethynyl)-3-hydroxy-cyclohexyl]-acetamide.
(±)-(1RS,3SR)-N-[3-(3-Fluoro-phenylethynyl)-3-hydroxy-cyclohexyl]-acetamide
(±)-(1RS,3SR)-[3-Hydroxy-3-(3-methoxy-phenylethynyl)-cyclohexyl]-carbamic acid ethyl ester
(±)-(1RS,3RS)-N-[3-Hydroxy-3-(3-methoxy-phenylethynyl)-cyclohexyl]-acetamide
(±)-(1RS,3SR)-N-[3-Hydroxy-3-(3-methoxy-phenylethynyl)-cyclohexyl]-acetamide.
(±)-(1RS,3RS)-[3-Hydroxy-3-(3-methoxy-phenylethynyl)-cyclohexyl]-carbamic acid *tert*-butyl ester
(±)-(1RS,3SR)-[3-Hydroxy-3-(3-methoxy-phenylethynyl)-cyclohexyl]-carbamic acid *tert*-butyl ester
(±)-[(4aRS,5SR,8aSR)-5-(3-chloro-phenylethynyl)-5-hydroxy-octahydro-quinolin-1-yl]-(4-methyl-piperazin-1-yl)-methanone
(±)-(4aRS,5RS,8aSR)-5-(3-chloro-phenylethynyl)-5-hydroxy-octahydro-quinoline-1-carboxylic acid ethyl ester and (±)-(4aRS,5SR,8aSR)- 5-(3-chloro-phenylethynyl)-5-hydroxy-octahydro-quinoline-1-carboxylic acid ethyl ester
(±)-(4aRS,5SR,8aSR)- 5-Hydroxy-5-m-tolylethynyl-octahydro-quinoline-1-carboxylic acid ethyl ester
(±)-(4aRS,5RS,8aSR)- 5-Hydroxy-5-m-tolylethynyl-octahydro-quinoline-1-carboxylic acid ethyl ester.

Further examples of mGluR, in particular mGluR5, modulators include compounds of the formula (I) as defined in WO 2004/014881 and compounds of the formula (I) as defined in WO 2007/021575.

Compounds of the disclosure and their pharmaceutically acceptable acid addition salts, hereinafter referred to as agents of the disclosure , exhibit valuable pharmacological properties and are therefore useful as pharmaceuticals.

Compounds of the disclosure may exhibit a marked and selective modulating, especially antagonistic, action at human mGluRs, in particular mGluR5s. This can be determined in vitro for example at recombinant human metabotropic glutamate receptors, especially PLC-coupled subtypes thereof such as mGluR5, using different procedures like, for example, measurement of the inhibition of the agonist induced elevation of intracellular Ca²⁺ concentration in accordance with L. P. Daggett et al., Neuropharm. Vol. 34, pages 871-886 (1995), P. J. Flor et al., J. Neurochem. Vol. 67, pages 58-63 (1996) or by determination to what extent the agonist induced elevation of the inositol phosphate turnover is inhibited as described by T. Knoepfel et al., Eur. J. Pharmacol. Vol. 288, pages 389-392 (1994), L. P. Daggett et al., Neuropharm. Vol. 67, pages 58-63 (1996) and references cited therein. Isolation and expression of human mGluR subtypes are described in US Patent No. 5,521,297. Selected agents of the invention show IC50 values for the inhibition of the agonist (e.g. glutamate or quisqualate) induced elevation of intracellular Ca2+ concentration or the agonist (e.g. glutamate or quisqualate) induced inositol phosphate turnover, measured in recombinant cells expressing hmGluR5a of about 1 nM to about 50 µM.

Compounds of the disclosure are useful in the treatment, prevention or delay of progression of Parkinson's Disease and disorders associated with Parkinson's Disease. Parkinson's Disease is a degenerative disorder of the central nervous system that often impairs the sufferer's motor skills and speech. Characteristics of Parkinson's Disease are varied and include one or more of the following: tremor, rigidity, bradykinesia, akinesia, gait and postural disturbances, postural instability, speech and swallowing disturbances and cognitive impairment e.g. memory loss, dementia and slowed reaction times. Compounds of the disclosure may be useful to treat, prevent or delay the progression of one or more of the characteristics of Parkinson's Disease. In one embodiment, the compounds of the disclosure are useful in the treatment, prevention or delay of progression of disorders which are associated with Parkinson's Disease. An example of such a disorder is Parkinson's dyskinesia e.g. Parkinson's Disease L-dopa induced dyskinesia. Parkinson's dyskinesia often, although not exclusively, occurs as a side-effect of treatment of Parkinson's disease with levodopa (L-dopa), a precursor of dopamine. Characteristics of Parkinson's dyskinesia include motor impairment, e.g. the appearance of slow and uncoordinated involuntary movements, shaking, stiffness and problems walking. Patients treated with L-dopa often have reduced symptoms of Parkinson's disease but they experience increasing difficulties to remain standing or even sitting. After prolonged use of L-dopa, a majority of patients develop dyskinesia.

Dyskinesia can occur at any time during the cycle of treatment with L-dopa. In one embodiment, the compounds of the disclosure are for the treatment of dyskinesia which occurs at the time of peak L-dopa plasma concentrations in the patient. In one embodiment, the compounds of the disclosure are for the treatment of dyskinesia which occurs when the L-dopa plasma concentrations in a patient rise or fall (diphasic dyskinesia).

Dyskinesia can also develop in Parkinson's disease sufferers who do not take L-dopa. In one embodiment, the compounds are for the treatment of non-L-dope induced Parkinson's dyskinesia.

Treatment may comprise a reduction in the characteristics associated with Parkinson's dyskinesia, including a reduction in the scale of involuntary movements, a reduction in the number of involuntary movements, an improvement in the ability to carry out normal tasks, an improved ability to walk, increased period of time between episodes of dyskinesia.

In the case of prophylactic treatment, the compounds of the disclosure may be used to delay or prevent the onset of Parkinson's dyskinesia.

The compounds may be useful in the treatment, prevention or delay of progression of dystonia, including primary and secondary dystonia. The dystonia may be, for example, neuroleptic or L-Dopa-induced. The compounds may also be useful in the treatment of other disorders associated with Parkinson's disease, including movement disorders such as tardive dyskinesia or tics.

In addition, the compounds may be useful in the treatment, prevention or delay of progression of Huntington's disease, Tourette's syndrome, Restless legs syndrome, Retts syndrome.

For the above-mentioned indications (the conditions and disorders) the appropriate dosage will vary depending upon, for example, the compound employed, the host, the mode of administration and the nature and severity of the condition being treated. However, in general, satisfactory results in animals are indicated to be obtained at a daily dosage of from about 0.01 to about 100 mg/kg body weight, preferably from about 0.1 to about 10 mg/kg body weight, e.g. 1 mg/kg. In larger mammals, for example humans, an indicated daily dosage is in the range from about 0.1 to about 1000 mg, preferably from about 1 to about 400 mg, most preferably from about 10 to about 100 mg of an mGluR, e.g. mGluR5, antagonist or other modulator conveniently administered, for example, in divided doses up to four times a day.

For use according to the invention, an mGluR modulator (e.g. an mGluR5 modulator, in particular an mGluR5 antagonist) may be administered as single active agent or in combination with other active agents, in any usual manner, e.g. orally, for example in the form of tablets or capsules, or parenterally, for example in the form of injection solutions or suspensions.

Moreover, the present disclosure provides a pharmaceutical composition comprising an mGluR modulator (e.g. an mGluR5 modulator, in particular an mGluR5 antagonist) in association with at least one pharmaceutical carrier or diluent for use in the treatment of Parkinson's Disease. In one embodiment, the composition is for use in the treatment of Parkinson's dyskinesia e.g. Parkinson's Disease L-dopa induced dyskinesia. Such compositions may be manufactured in conventional manner. Unit dosage forms may contain, for example, from about 2.5 to about 25 mg of one or more mGluR modulator, e.g. mGluR5 antagonist or other modulator.

A pharmaceutical composition of the disclosure may further comprise L-Dopa. The composition may further comprise a Dopa decarboxylase inhibitor, e.g. benserazide.

The usefulness of the compounds of the disclosure in the treatment of the above-mentioned disorders can be confirmed in a range of standard tests including those indicated below:

The pharmaceutical compositions according to the disclosure are compositions for enteral, such as nasal, rectal or oral, or parenteral, such as intramuscular or intravenous, administration to warm-blooded animals (human beings and animals) that comprise an effective dose of the pharmacological active ingredient alone or together with a significant amount of a pharmaceutically acceptable carrier. The dose of the active ingredient depends on the species of warm-blooded animal, body weight, age and individual condition, individual pharmacokinetic data, the disease to be treated and the mode of administration.

The pharmaceutical compositions comprise from approximately 1% to approximately 95%, preferably from approximately 20% to approximately 90%, active ingredient. Pharmaceutical compositions according to the disclosure may be, for example, in unit dose form, such as in the form of ampoules, vials, suppositories, dragées, tablets or capsules.

The pharmaceutical compositions of the present disclosure are prepared in a manner known per se, for example by means of conventional dissolving, lyophilizing, mixing, granulating or confectioning processes. Such processes are exemplified in WO 2005/079802, WO 2003/047581, WO 2004/000316, WO 2005/044265, WO 2005/044266, WO 2005/044267, WO 2006/114262 and WO 2007/071358.

The disclosure also provides a product, for example a kit, comprising an mGluR modulator and L-Dopa as a combined preparation for simultaneous, separate or sequential use in therapy. The product may further comprise a Dopa decarboxylase inhibitor, e.g. benserazide.

The action of mGluR modulatos, e.g. mGluR anatagonists on Parkinson's Disease and associated disorders e.g. Parkinson's dyskinesia, for example, Parkinson's Disease levodopa (L-dopa) induced Parkinson's dyskinesia as described herein, may be conducted in the following way.

Firstly, it has been found through imaging techniques that the compounds of the present disclosure are able to penetrate the brain and bind to mGluR receptors, in particular mGluR5 receptors. Secondly, it has been observed that patients taking a compound, such as an mGluR modulators as described herein have shown an increase in cognition.

Clinical testing of the compounds as mentioned herein may be conducted, for example, in one of the following study designs. The skilled physician may look at a number of aspects of a patients behavious and abilities.The skilled person will of course realise that such studies are considered as guidelines and the certain aspects of the studies may be modified and redefined depending on the circumstance and environment, for example.

### Clinical Design: Improvement Trials

### Trial A: Normal Patient Population

A patient population, with a normal control is dosed once a day for a week or longer tested. The test is designed to allow for improvement, I.e. that there is a measurable parameter increase of the impaired function The patients are tested at the beginning and at the end of the dosage period and the results are compared and analysed.

### Trial B: Deficit population

A patient population with a deficit associated with Parkinson's Disease and associated disorders e.g. Parkinson's dyskinesia, for example, Parkinson's Disease levodopa (L-dopa) induced Parkinson's dyskinesia is dosed once a day for a week or longer and tested. The test is designed to allow for improvement, I.e. that there is a measurable parameter increase of the impaired function. The patients are tested at the beginning and at the end of the dosage period and the results are compared and analysed.

### Considerations for designing a trial

- When designing a trial, the skilled person will appreciate the need to protect both against floor and ceiling effects. In other words, the study designing should allow cognition to the measurably raised or lowered.
- Conditions that artificially impair a function, e.g. cognition, are one way to test enhancement of that function. Such conditions are, for example, sleep deprivation and pharmacological challenges.
- Placebo control is required for all trials.
- In assessing the data, evaluation of the likelihood of learning and practice effects from repeat assessments must be made. The likelihood of such effects contaminating the data to produce false positives should be taken in to account when designing the test, e.g. the tests should not be identical (e.g. commit the same list of words to memory) but designed to study the same mechanism. Other countermeasures may include single testing at the end of a trial only.

### Example 1: Assessment of antidyskinetic effect of Compound A, a selective mGluR5 antagonist, in parkinsonian primates

### Method

Six female ovariectomized cynomolgus monkeys (Macaca fascicularis) weighing between 2.8- 4.4 kg were used in the assessment. The animals were rendered parkinsonian by continuous infusion of 1-methyl-4-phenyl-1,2,3,6-tetrahydropyridine (MPTP, Sigma-Aldrich, Canada, Oakville, Ontario) using subcutaneous osmotic minipumps (Alzet, 0.5 mg/24h) until they developed a stable parkinsonian syndrome. After 1 to 3 months of recuperation, animals were treated daily with L-Dopa 100/25 capsule p.o. (Prolopa, Hoffmann-La Roche; a mixture of 100 mg of L-Dopa and 25 mg benserazide) until clear and reproducible dyskinesias developed.

### Assessment

Monkeys were observed through a one-way screen window in their home cage. They were observed and scored repeatedly at baseline and after a standard s.c. dose of L-DOPA methyl ester always with benserazide. Locomotor activity was assessed and followed with an electronic monitoring system (Data Science). Antiparkinsonian responses were evaluated by measuring the locomotor activity and a Parkinson disability scale (see Hadj Tahar A et al, Clin Neuropharmacol 2000; 23:195-202; and Samadi P et al, Neuropharmacology 2003; 45:954-963). Dyskinesias were closely monitored and scored according to a dyskinesia rating scale (also described in Hadj Tahar A et al; and Samadi P et al) every 15 minutes until the end of the effect. The doses of L-DOPA methyl ester and benserazide were chosen to induce motor activation and reproducible dyskinesia but no excessive agitation (15-30 mg/kg/50 mg).

### Protocol

Monkeys were observed for at least two hours following an oral administration of vehicle (Klucel). On a subsequent day, the dose of L-DOPA methyl ester/benserazide s.c. selected was tested once. The animals were observed (with measures of parkinsonian and dyskinetic scores) for the entire duration of the L-DOPA effect and were also monitored for locomotor activity. This provided vehicle control values as well as L-DOPA antiparkinsonian and dyskinesia response data for comparison with combinations of Compound A and L-DOPA.

The monkeys were then tested with four doses of the selective mGluR5 antagonist Compound A (5, 25, 125 and 250 mg/kg) in combination with a fixed s.c. dose of L-DOPA methyl ester/benserazide. A suspension for oral administration of Compound A (Suspension in Klucel HF) was administered one hour before L-DOPA methyl ester. After each dose, the animals were observed (with measures of parkinsonian and dyskinetic scores) for the entire duration of effect and monitored for locomotor activity or any change in behaviour (e.g. circling, excitement, lethargy and sleepiness). Three days were left between each mGluR5 antagonist dose investigated.

### Results

It was found that co-treatment of a mGluR5 antagonist with L-Dopa significantly reduced L-Dopa induced dyskinesias whilst maintaining antiparkinsonian activity. In particular, the L-Dopa induced dyskinesias scores were significantly decreased with the addition of Compound A at doses of 25, 125 and 250 mg/kg.

### Example 2: Assessment of antidyskinetic effect of repeated administration of Compound A, a selective mGluR5 antagonist, in parkinsonian primates

### Method

Six female ovariectomized cynomolgus monkeys (Macaca fascicularis) weighing between 2.8- 4.4 kg were used in the assessment. The animals were rendered parkinsonian by continuous infusion of 1-methyl-4-phenyl-1,2,3,6-tetrahydropyridine (MPTP, Sigma-Aldrich, Canada, Oakville, Ontario) using subcutaneous osmotic minipumps (Alzet, 0.5 mg/24h) until they developed a stable parkinsonian syndrome. After 1 to 3 months of recuperation, animals were treated daily with L-Dopa 100/25 capsule p.o. (Prolopa, Hoffmann-La Roche; a mixture of 100 mg of L-Dopa and 25 mg benserazide) until clear and reproducible dyskinesias developed.

### Assessment

Monkeys were observed through a one-way screen window in their home cage. They were observed and scored repeatedly at baseline and after a standard s.c. dose of L-Dopa methyl ester always with benserazide. Locomotor activity was assessed and followed with an electronic monitoring system (Data Science). Antiparkinsonian responses were evaluated by measuring the locomotor activity and a Parkinson disability scale (see Hadj Tahar A et al, Clin Neuropharmacol 2000; 23:195-202; and Samadi P et al, Neuropharmacology 2003; 45:954-963). Dyskinesias were closely monitored and scored according to a dyskinesia rating scale (also described in Hadj Tahar A et al; and Samadi P et al) every 15 minutes until the end of the effect. The intermediate doses of L-Dopa methyl ester/benserazide range from 20-30 mg/kg/ 50 mg and the low doses of L-Dopa methyl ester/benserazide range from 5-15 mg/kg/ 50 mg.

### Protocol

At least two weeks before the study, all animals received an L-dopa capsule p.o. 3 times per week to achieve good priming.

During each week of the study, the animals were observed (with measures of parkinsonian and dyskinetic scores) for the entire duration of effect and monitored for locomotor activity or any change in behaviour (e.g. circling, excitement, lethargy and sleepiness).

During the first week of the study (7 consecutive days), the monkeys were tested with daily s.c. injections of the intermediate doses of L-Dopa methyl ester.

During the second week of the study (7 consecutive days), the monkeys were tested with daily suspensions of 25 mg/kg of Compound A one hour before the administration of the daily intermediate doses of L-Dopa.

During the third week of the study (7 consecutive days), the monkeys were tested with daily s.c. injections of the intermediate doses of L-Dopa methyl ester, until the response returned to the initial level observed during the first week of testing.

During the fourth week of the study (7 consecutive days), the monkeys were tested once every three days with daily s.c. injections of low doses of L-Dopa methyl ester. Every experimental day was separated by the administration of intermediate doses of L-Dopa methyl ester in order to maintain priming.

During the fifth week of the study (7 consecutive days), the monkeys were tested with daily suspensions of 25 mg/kg of Compound A in addition to the low dose L-Dopa methyl ester administration once every three days. Compound A was administered one hour before the low dose L-Dopa methyl ester administration.

During the sixth week of the study (7 consecutive days), the monkeys were tested once every three days with low dose L-Dopa, until the response returned to the initial level observed during the first week of testing.

### Results

It was found that repeated administration of a mGluR5 antagonist following acute administration with intermediate dose L-Dopa significantly reduced L-Dopa induced dyskinesias whilst maintaining antiparkinsonian activity. In particular, the L-Dopa induced dyskinesias scores were significantly decreased with the addition of Compound A at a dose of 25 mg/kg.

It was found that administration of a mGluR5 antagonist potentiated the antiparkinsonian response of a low dose L-Dopa. In particular, the antiparkinsonian response of low dose L-Dopa was potentiated with the repeated addition of Compound A at a dose of 25 mg/kg.

## Claims

1. (-)-(3aR,4S,7aR)-4-Hydroxy-4-m-tolylethynyl-octahydro-indole-1-carboxylic acid methyl ester in free base or acid addition salt form, for use in the treatment, prevention or delay of progression of a Parkinson's associated levodopa (L-dopa) induced dyskinesia.

2. A pharmaceutical composition comprising (-)-(3aR,4S,7aR)-4-Hydroxy-4-m-tolylethynyl-octahydro-indole-1-carboxylic acid methyl ester as defined in claim 1 for use in the treatment, prevention or delay of progression of Parkinson's disease associated levodopa (L-dopa) induced dyskinesia.

3. A composition for use according to claim 2, which further comprises levodopa (L-dopa).

4. A kit comprising (-)-(3aR, 4S,7aR)-4-Hydroxy-4-m-tolylethynyl-octahydro-indole-1-carboxylic acid methyl ester as defined in any of claims 1 to 3 and instructions, wherein said kit is for use in the treatment, prevention or delay of progression of Parkinson's associated levodopa (L-dopa) induced dyskinesia.

5. A product comprising (-)-(3aR,4S,7aR)-4-Hydroxy-4-m-tolylethynyl-octahydro-indole-1-carboxylic acid methyl ester as defined in any one of claims 1 to 4 and levodopa (L-dopa) as a combined preparation for simultaneous, separate or sequential use in the treatment, prevention or delay of progression of Parkinson's associated levodopa (L-dopa) induced dyskinesia.

## Patentansprüche

1. (-)-(3aR,4S,7aR)-4-Hydroxy-4-m-tolylethinyloctahydroindol-1-carbonsäuremethylester in Form der freien Base oder des Säureadditionssalzes zur Verwendung bei der Behandlung, Prävention oder Verzögerung der Progression einer Parkinson-assoziierten Levodopa (L-Dopa)-induzierten Dyskinesie.

2. Pharmazeutische Zusammensetzung, die (-)-(3aR,4S,7aR)-4-Hydroxy-4-m-tolylethinyloctahydroindol-1-carbonsäuremethylester nach Anspruch 1 zur Verwendung bei der Behandlung, Prävention oder Verzögerung der Progression einer Parkinson-assoziierten Levodopa (L-Dopa)-induzierten Dyskinesie umfasst.

3. Zusammensetzung zur Verwendung nach Anspruch 2, die weiter Levodopa (L-Dopa) umfasst.

4. Kit, das (-)-(3aR,4S,7aR)-4-Hydroxy-4-m-tolylethinyloctahydroindol-1-carbonsäuremethylester nach einem der Ansprüche 1 bis 3 und Anweisungen umfasst, wobei das Kit zur Verwendung bei der Behandlung, Prävention oder Verzögerung der Progression einer Parkinson-assoziierten Levodopa (L-Dopa)-induzierten Dyskinesie ist.

5. Produkt, das (-)-(3aR,4S,7aR)-4-Hydroxy-4-m-tolylethinyloctahydroindol-1-carbonsäuremethylester nach einem der Ansprüche 1 bis 4 und Levodopa (L-Dopa) als Kombinationspräparat zur gleichzeitigen, getrennten oder aufeinanderfolgenden Verwendung bei der Behandlung, Prävention oder Verzögerung der Progression einer Parkinson-assoziierten Levodopa (L-Dopa)-induzierten Dyskinesie umfasst.

## Revendications

1. Ester méthylique de l'acide (-)-(3aR, 4S, 7aR)-4-hydroxy-4-m-tolyléthynyl-octahydro-indole-1-carboxylique sous forme de base libre ou de sel d'addition d'acide, pour une utilisation dans le traitement, la prévention ou le retardement de la progression de la dyskinésie induite par la lévodopa (L-dopa) associée à la maladie de Parkinson.

2. Composition pharmaceutique comprenant de l'ester méthylique de l'acide (-)-(3aR, 4S, 7aR)-4-hydroxy-4-m-tolyléthynyl-octahydro-indole-1-carboxylique selon la revendication 1, pour une utilisation dans le traitement, la prévention ou le retardement de la progression de la dyskinésie induite par la lévodopa (L-dopa) associée à la maladie de Parkinson.

3. Composition pour une utilisation selon la revendication 2, qui comprend en outre de la lévodopa (L-dopa).

4. Kit comprenant de l'ester méthylique de l'acide (-)-(3aR, 4S, 7aR)-4-hydroxy-4-m-tolyléthynyl-octahydro-indole-1-carboxylique selon l'une quelconque des revendications 1 à 3, et des instructions, dans lequel ledit kit est prévu pour une utilisation dans le traitement, la prévention ou le retardement de la progression de la dyskinésie induite par la lévodopa (L-dopa) associée à la maladie de Parkinson.

5. Produit comprenant de l'ester méthylique de l'acide (-)-(3aR, 4S, 7aR)-4-hydroxy-4-m-tolyléthynyl-octahydro-indole-1-carboxylique selon l'une quelconque des revendications 1 à 4, et de la lévodopa (L-dopa) en une préparation combinée pour une utilisation simultanée, séparée ou séquentielle dans le traitement, la prévention ou le retardement de la progression de la dyskinésie induite par la lévodopa (L-dopa) associée à la maladie de Parkinson.
